# EUROPEAN PATENT SPECIFICATION

(11) **EP 0 444 639 B1**
(45) Date of publication and mention of the grant of the patent: **31.01.1996**
(21) Application number: 91102936.1
(22) Date of filing: 27.02.1991
(51) Int. Cl.: C12N 15/55, C12N 9/80, C12P 13/02

(54) **A gene encoding a polypeptide having nitrile hydratase activity, a transformant containing the gene and a process for the production of amides using the transformant**
Gen, das ein Polypeptid mit Nitrilhydratase-Aktivität codiert, eine dieses Gen enthaltende Transformante und Verfahren zur Herstellung von Amiden mit dieser Transformante.
Gène codant pour une polypeptide avec une activité nitrile hydratase, transformant contenant un gène et procédé de production d'amides avec ce transformant

(30) Priority: 28.02.1990 JP 48079/90
(43) Date of publication of application: 04.09.1991
(73) Proprietor: NITTO CHEMICAL INDUSTRY CO., LTD., Tokyo (JP); Beppu, Teruhiko, Horinouchi Suginami-ku, Tokyo (JP); YAMADA, Hideaki, Kyoto-shi Kyoto-Fu (JP)
(72) Inventor: Beppu, Teruhiko, Tokyo (JP); Yamada, Hideaki, Sakyo-ku Kyoto-shi Kyoto-fu (JP); Nagasawa, Toru, Sakyo-ku Kyoto-shi Kyoto-fu (JP); Horinouchi, Sueharu, Koutou-ku Tokyo (JP); Nishiyama, Makoto, Shinjuku-ku Tokyo (JP)
(74) Representative: VOSSIUS & PARTNER

(56) References cited:
- EP-A- 0 093 782
- FR-A- 2 633 938
- US-A- 4 637 982
- EUROPEAN JOURNAL OF BIOCHEMISTRY vol. 162, no. 3, February 1987, BERLIN, DE pages 691 - 698; NAGASAWA, T. ET AL.: 'Nitrile hydratase of Pseudomonas chlororaphis B23; purification and characterization'
- AGRICULTURAL AND BIOLOGICAL CHEMISTRY. vol. 46, no. 5, May 1982, TOKYO JP pages 1183 - 1189; ASANO, Y. ET AL.: 'A new enzymatic method of acrylamide production'
- AGRICULTURAL AND BIOLOGICAL CHEMISTRY. vol. 50, no. 11, November 1986, TOKYO JP pages 2859 - 2865; YAMADA, H. ET AL.: 'Optimum culture conditions for production by Pseudomonas chlororaphis B23 of nitrile hydratase'
- M. Kobayashi et al., TIBTECH NOVEMBER 1992, vol. 10, pp. 402-408

## Description

The present invention relates to a gene which is derived from Pseudomonas chlororaphis B23 and which encodes a polypeptide having nitrile hydratase activity to hydrate nitriles to amides. The invention also relates to a recombinant DNA containing the gene, and a transformant transformed with the recombinant DNA. The present invention further relates to a method of the production of nitrile hydratase using the transformant and of amides using nitrile hydratase.

Nitrile hydratase or nitrilase is known as an enzyme that hydrates nitriles to amides. Microorganisms that produce nitrile hydratase include those belonging to the genus Bacillus, the genus Bacteridium, the genus Micrococcus and the genus Brevibacterium (See, JP-B-62-21517/1989, USP No. 4,001,081), the genus Corynebacterium and the genus Nocardia (See, JP-B-56-17918/1989, USP No. 4,248,968), the genus Pseudomonas (See, JP-B-59-37951/1984, USP No. 4,637,982), the genus Rhodococcus, the genus Arthrobacter and the genus Microbacterium (See, JP-A-61-162193/1986, EP-A-0188316), and Rhodococcus rhodochrous (See, JP-A-2-470/1990, EP-A-0307926).

Nitrile hydratase has been used to hydrate nitriles to amides. In the invention, microorganisms are engineered to contain multiple copies of a recombinant DNA encoding nitrile hydratase according to a recombinant DNA technology. The recombinant produces a remarkably high level of nitrile hydratase compared with conventional methods.

The present inventors previously disclosed a gene derived from Rhodococcus sp. N-774 (FERM BP-1936) which also encodes a polypeptide having nitrile hydratase activity (JP-A-2-119778/1988).

In contrast, the present inventors utilized a gene derived from Pseudomonas chlororaphis B23 described in said USP No. 4,637,982 for the production of nitrile hydratase. We isolated the gene encoding nitrile hydratase, inserted the gene into a suitable plasmid vector and transformed an appropriate host with the recombinant plasmid, thus successfully obtained the transformant producing nitrile hydratase.

### The present invention relates to

(1) a gene encoding a polypeptide which has nitrile hydratase activity and which comprises the α-subunit of the amino acid sequence as defined in the Sequence Listing by SEQ ID: No. 1, and the β-subunit of the amino acid sequence as defined in the Sequence Listing by SEQ ID: No. 2;
(2) a gene as described in (1) encoding α- and β-subunits, comprising the α-subunit-encoding sequence as defined in the Sequence Listing by SEQ ID: No. 3, and the β-subunit-encoding sequence as defined in the Sequence Listing by SEQ ID: No. 4;
(3) a recombinant DNA comprising a vector containing the gene as described in (1) or (2);
(4) a transformant transformed with the recombinant DNA as described in (3);
(5) a method for the production of nitrile hydratase which comprises culturing the transformant as described in (4) and recovering nitrile hydratase from the culture;
(6) a method for the production of amides which comprises hydrating nitriles using the nitrile hydratase as described in (5) to form amides; and
(7) a method for the production of amides which comprises culturing the transformant as described in (4), and hydrating nitriles using the resultant culture, isolated bacterial cells, treated matter thereof, or a fixed material of them, to form amides.

The present invention is described in detail as follows.

### (1) Isolation and Purification of Nitrile Hydratase and Partial Amino Acid Sequencing of Nitrile Hydratase

Nitrile hydratase is isolated and purified from Pseudomonas chlororaphis B23 and separated into α and β subunits using HPLC. N-Terminal amino acid sequence of the subunits is determined and shown in the Sequence Listing by SEQ ID: No. 5 and No. 6, respectively.

### (2) Preparation of a DNA Probe for a Nitrile Hydratase Gene

A DNA probe is prepared from JM105/pYUK121 (FERM BP-1937) as described in JP-A-2-119778/1990 due to the high degree of homology in the amino acid sequence between the nitrile hydratase β subunit of Rhodococcus sp. N-774 described in said Japanese Patent official gazette and that of Pseudomonas chlororaphis B23. Plasmid pYUK121 containing nitrile hydratase gene derived from Rhodococcus sp. N-774 is prepared from a JM105/pYUK121 culture. pYUK121 DNA is digested with SphI and SalI. The SphI-SalI fragment contains the nitrile hydratase gene (shown in the Sequence Listing as defined by SEQ ID: No. 7) of Rhodococcus sp. N-774. The DNA fragment is radiolabeled to give a probe.

### (3) Detection of a DNA Segment Containing a Nitrile Hydratase Gene from the Chromosome of Pseudomonas chlororaphis B 23

Chromosomal DNA is prepared from a Pseudomonas chlororaphis B23 culture. Chromosomal DNA is digested with restriction enzymes and hybridized to the probe described in (2), according to the Southern hybridization method [Southern, E.M., J. Mol. Biol. 98, 503 (1975)]. Two DNA fragments of different length are screened.

### (4) Construction of a Recombinant Plasmid

A recombinant plasmid is constructed by inserting the chromosomal DNA fragment as prepared in (3) into a plasmid vector.

### (5) Transformation and Screening for a Transformant Containing the Recombinant Plasmid

Transformants are prepared using the recombinant plasmid as described in (4). The transformant containing the recombinant plasmid is selected using the probe as described in (2) according to the colony hybridization method [R. Bruce Wallace et al., Nuc. Aci. Res. 9, 879 (1981)]. Additionally, the presence of the nitrile hydratase gene in the recombinant plasmid is confirmed using the Southern hybridization method. The plasmids thus selected are designated as pPCN1 and pPCN3.

### (6) Isolation and Purification of Plasmid DNA and Construction of the Restriction Map

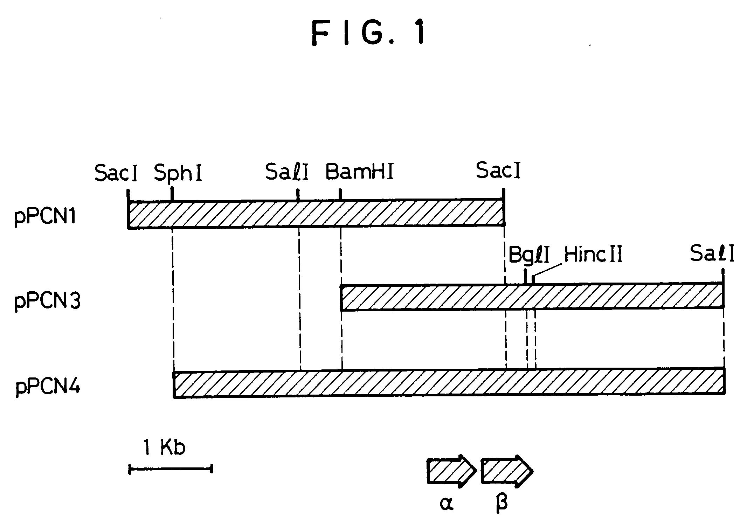

Plasmid DNAs of pPCN1 and pPCN3 as described in (5) are isolated and purified. The restriction maps of the DNAs are constructed (Fig. 1) to determine the region containing nitrile hydratase gene.

### (7) DNA Sequencing

The extra segment of the inserted DNA fragment in pPCN1 and pPCN3 is excised using an appropriate restriction enzyme. The inserted DNA fragment is then used for sequencing. The nucleotide sequence of the DNA fragment (see the Sequence Listing as defined by SEQ ID: No. 8) reveals that it contains the sequence deduced from the amino acid sequence as described in (1).

### (8) Insertion of the DNA Fragment into an Expression Vector and Transformation

The DNA fragment is cleaved from pPCN1 and pPCN3 using appropriate restriction enzymes. These two fragments are ligated and inserted into the expression vector pUC19. The construct is used for transformation of E. coli JM105 (Amersham), and the transformant is designated as JM105/pPCN4.

### (9) Production of Nitrile Hydratase Using the Transformant and Conversion of Nitriles to Amides

The transformant as described in (8) is cultured. The bacterial cells are mixed with nitriles, a substrate of nitrile hydratase, and amides are produced.

Pseudomonas chlororaphis B23 was deposited with Fermentation Research Institute, Agency of Industrial Science and Technology and was assigned the accession number FERM BP-187. A transformant JM105/pPCN4 was deposited with the same and assigned the accession number FERM BP-2779.

Any vector including a plasmid vector (e.g., pAT153, pMP9, pHC624, pKC7, etc.), a phage vector (e.g., λgt11 (Toyobo), Charon 4A (Amersham), etc.) may be used for the present invention. Enzymes which may be used include SphI, SalI, SacI, BamHI, EcoRI, PstI and the like, which are commercially available (Takara Shuzo). Various hosts may be used for transformation including but not limited to E. coli JM105 and TGl. Culture media for the transformant are those ordinarily used in the art.

Conversion of nitriles to amides is carried out using nitrile hydratase, crude nitrile hydratase, the culture of the transformant, the isolated bacterial cells or treated matter thereof, and the like, prepared from the culture of the transformant.

Suitable nitriles include those having 2-4 carbon atoms such as acetonitrile, propionitrile, acrylonitrile, methacrylonitrile, n-butyronitrile and isobutyronitrile, and acrylonitrile is preferred.

Fig. 1 shows restriction maps of recombinant plasmids, pPCN1, pPCN3 and pPCN4.

The present invention discloses the amino acid sequence and nucleotide sequence of the α- and β-subunits of nitrile hydratase derived from Pseudomonas chlororaphis B23. The gene encoding nitrile hydratase is inserted into an expression vector and the recombinant vector is used for transformation. The transformant contains multiple copies of the gene and produces a higher level of nitrile hydratase than conventionally used microorganisms.

The present invention is illustrated by the following Example.

The following abbreviations are used in the Example.
- TE:: Tris-HCl (10 mM; pH 7.8), EDTA (1 mM, pH 8.0)
- TNE:: Tris-HCl (50 mM; pH 8.0), EDTA (1 mM, pH 8.0), NaCl (50 mM)
- STE:: Tris-HCl (50 mM; pH 8.0), EDTA (5 mM, pH 8.0), Sucrose (35 mM)
- 2xYT medium:: 1.6% Trypton; 1.0% Yeast extract, 0.5% NaCl

### Example

### (1) Isolation and Purification of Nitrile Hydratase and the Amino Acid Sequencing of a Portion of Nitrile Hydratase

Pseudomonas chlororaphis B23 was cultured in a medium (10 g/ℓ of sucrose, 4 g/ℓ of methacrylamide, 0.5 g/ℓ of KH₂PO₄, 0.5 g/ℓ of K₂HPO₄, 0.5 g/ℓ of MgSO₄·7H₂O, 0.01 g/ℓ of FeSO₄·7H₂O, pH 7.0) at 25°C for 28 hours. The bacterial cells were harvested. 100 g of the bacterial cells was disrupted and fractionated with ammonium sulfate. The sample was dialyzed and the dialysate was centrifuged. The supernatant was removed and loaded on DEAE-Sephacel chromatography, Octyl-Sepharose CL-4B chromatography, Phenyl-Sepharose CL-4B chromatography and Sephadex G-150 chromatography. Active fractions were collected and dialyzed. The dialysate containing the enzyme was loaded on a high performance liquid chromatography using a reversed phase column (Senshu Pak VP-304-1251, Senshu Kagaku), and two subunits (α and β) were obtained. The N-terminal amino acid sequence of α- and β-subunits was determined using an amino acid sequencer (Applied Biosystems model 470A). N-terminal amino acid sequences of α- and β-subunits are shown in the Sequence Listing as defined by SEQ ID: No. 5 and SEQ ID: No. 6 respectively.

### (2) Preparation of a DNA Probe for Nitrile Hydratase Gene

E. coli JM105 containing pYUK121 (FERM BP-1937) was cultured in 100 ml of 2×YT medium containing 50 µg/ml of ampicillin at 30°C overnight (12 hours). The bacterial cells were harvested and TNE was added to the cells. The cell suspension was then centrifuged. 8 ml of STE and 10 mg of lysozyme were added to the pellet. The mixture was incubated at 0°C for five minutes followed by the addition of 4 ml of 0.25M EDTA. 2 ml of 10% SDS and 5 ml of 5M NaCl were then added to the mixture at room temperature. The resultant mixture was left standing at 0-4°C for three hours and then ultracentrifuged. 1/2 volume of 30% PEG 6000 was added to the supernatant. The mixture was allowed to stand at 0-4°C overnight (12 hours) and centrifuged. TNE was added to the pellet to bring the volume to 7.5 ml and CsCl was then added to the suspension. The mixture was centrifuged to remove proteins. Then, 300-500 mg/ml of ethidium bromide was added to the supernatant. The mixture was transferred to a centrifuge tube. The tube was heat-sealed and then ultracentrifuged. cccDNA was extracted using a peristaltic pump. A bit more than equal volume of isopropyl alcohol saturated with water was added to the extract to remove ethidium bromide. The sample was dialyzed against TE. About 3 ml of purified pYUK121 was obtained.

pYUK121 DNA was digested with SphI and SalI, resulting in a 2.07 kb DNA fragment containing a nitrile hydratase gene derived from Rhodococcus sp. N-774. The fragment was radiolabeled with ³P to produce a probe. The nucleotide sequence of the probe is shown in the Sequence Listing as defined by SEQ ID: No. 7.

### (3) Preparation of a DNA Fragment Containing a Nitrile Hydratase Gene of Chromosome

Pseudomonas chlororaphis B23 was cultured in 100 ml of the medium as described in (1). The bacterial cells were harvested and washed with TNE. The pellet was then suspended in 10 ml of TE. 4 ml of 0.25M EDTA, 10-20 mg of lysozyme, 10-20 mg of achromoprotease and 10 ml of 10% SDS were added to the suspension. The mixture was incubated at 37°C for three hours. 15 ml of phenol was added to the mixture. The mixture was incubated at room temperature for 15 minutes and then centrifuged. To 15 ml of the upper layer, 0.7 ml of 2.5M sodium acetate and diethyl ether were added and the mixture was centrifuged. The upper layer was discarded. Two volumes of ethanol were added to the bottom layer and DNA was removed with a glass rod. DNA was rinsed with TE:ethanol 2:8, 1:9, and 0:10 (v/v) for five minutes each. DNA was then resuspended in 2-4 ml of TE (37°C). 10 µℓ of a mixture of RNase A and T1 was added to the suspension and the mixture was incubated at 30°C . An equal volume of phenol was added to the mixture. The mixture was then centrifuged. After centrifugation 2-4 ml of the upper layer was removed. More than equal amount of ether was added to the removed portion. The mixture was centrifuged. After centrifugation, the upper layer was discarded. The bottom layer was dialyzed overnight against 2 ℓ of TE containing a small amount of chloroform and further dialyzed against fresh TE for 3-4 hours. 4ml of crude chromosomal DNA was obtained. Enzymatic digestion of chromosomal DNA was carried out as follows:
(a) 2 µℓ of SacI + 3 µℓ of reaction buffer (10×) + 15 µℓ of chromosomal DNA + 10 µℓ of TE
(b) 2 µℓ of BamHI + 2 µℓ of SalI + 3 µℓ of reaction buffer (10×) + 15 µℓ of chromosomal DNA + 8 µℓ of TE

The mixtures were incubated at 37°C for an hour and electrophoresed on an agarose gel at 60 V for three hours. The Southern hybridization of chromosomal DNA was carried out using the probe as described in (2). About 4.6 kb and 4.7 kb fragments were found to show a strong hybridization.

15 µℓ of chromosomal DNA was digested with SacI, and BamHI and SalI and electrophoresed on an agarose gel as described above. 4.6 kb and 4.7 kb DNA fragments were cut out from the gel and placed in three volumes of 8M NaClO₄. The solution was dotted on a GF/C (Whatman) filter paper (6 mm in diameter). Ten drops (≃ 100 µℓ) of TE containing 6M NaClO₄ and then ten drops (≃ 100 µℓ) of 95% ethanol were added to the filter paper. The paper was air-dried and placed in 0.5 ml Eppendorf tube. 40 µℓ of TE was added to the tube and the tube was incubated at 37°C for 30 minutes. The tube was then centrifuged. About 40 µℓ of the supernatant was obtained which contained 4.6 kb and 4.7 kb DNA fragments containing a nitrile hydratase gene of chromosomal DNA.

### (4) Insertion of the Chromosomal DNA Fragment into a Vector

### (a) 4.6 kb DNA Fragment

2 µℓ of SacI, 3 µℓ of reaction buffer (10×) and 10 µℓ of TE were added to 10 µℓ of pUC18 DNA. The mixture was incubated at 37°C for an hour. 2 µℓ of 0.25M EDTA was added to the mixture to stop the reaction. Then 7 µℓ of 1M Tris-HCl (pH 9) and 3 µℓ of BAP (bacterial alkaline phosphatase) were added to the mixture. The mixture was incubated at 65°C for an hour. TE was then added to the mixture to bring the volume to 100 µℓ. The mixture was extracted 3× with an equal volume of phenol. An equal volume of ether was added to the extract. The bottom layer was removed and 10 µℓ of 3M sodium acetate and 250 µℓ of ethanol were added to the bottom layer. The mixture was incubated at -80°C for 30 minutes, centrifuged, dried, and resuspended in TE.

5 µℓ of pUC18 DNA thus obtained and 40 µℓ of the 4.6 kb DNA fragment as described in (3) were mixed. 6 µℓ of ligation buffer, 6 µℓ of ATP (6 mg/ml) and 3 µℓ of T4 DNA ligase were added to the mixture. The mixture was incubated at 4°C overnight (12 hours) to produce the recombinant plasmid containing the 4.6 kb DNA fragment in the SacI site of pUC18.

### (b) 4.7 kb DNA Fragment

pUC18 DNA was digested with BamHI and SalI. The 4.7 kb DNA fragment was inserted into the BamHI-SalI site of pUC18 in a similar manner as described in (4)-(a). The recombinant plasmid containing the 4.7 kb DNA fragment in the BamHI-SalI site of pUC18 was obtained.

### (5) Transformation and Screening of Transformants

E. coli JM105 (Amersham) was inoculated into 10 ml of 2xYT medium and incubated at 37°C for 12 hours. After incubation, the culture was added to a fresh 2×YT medium to a final concentration of 1%, and the mixture was incubated at 37°C for two hours. The culture was centrifuged and the pellet was suspended in 5 ml of cold 50 mM CaCl₂. The suspension was placed on 0°C for 40 minutes and then centrifuged. 0.25 ml of cold 50 mM CaCl₂ and 60 µℓ each of the recombinant plasmids as prepared in (4)(a) and (b) were added to the pellet in a separate tube. The mixture was incubated on ice for 40 minutes, heat-shocked at 42°C for two minutes, placed on ice for five minutes, and added to 10 ml of 2×YT medium. The mixture was incubated at 37°C for 90 minutes with shaking, then centrifuged. The pellet was suspended in 1 ml of 2×YT medium, and 10 µℓ of the suspension was plated on a 2×YT agar plate containing 50 µg/ml of ampicillin. The plate was incubated at 37°C. The colony grown on the plate was selected by the colony hybridization method: The colony was transferred to a nitrocellulose filter and lysed. The DNA fixed on the filter was hybridized to the probe as described in (2). The filter was autoradiographed and a recombinant colony was selected. Additionally, the presence of a nitrile hydratase gene in the transformant was confirmed according to the Southern hybridization method.

### (6) Isolation and Purification of Recombinant Plasmid and Construction of the Restriction Map of the Inserted DNA Fragments

The transformant selected as described in (5) was grown in 100 ml of a 2xYT medium containing 50 µg/ml of ampicillin at 37°C overnight (12 hours). The bacterial cells were harvested and TNE was added to the cells. The cells were collected again by centrifugation, and 8 ml of STE and 10 mg of lysozyme were added to the cells. The mixture was incubated at 0°C for five minutes. 4 ml of 0.25M EDTA, 2 ml of 10% SDS (at room temperature) and 5 ml of 5M NaCl were added to the mixture. The mixture was incubated at 0-4°C for three hours, and ultracentrifuged. 1/2 volume of 30% PEG 6000 was added to the supernatant. The mixture was incubated at 0-4°C overnight (12 hours) and centrifuged again. TNE was added to the pellet to bring the volume up to 7.5 ml. CsCl was added to the mixture. The mixture was centrifuged to rid of proteins. Then, 300-500 mg/ml of ethidium bromide was added to the supernatant and the mixture was transferred to a centrifuge tube. The tube was heat-sealed and ultracentrifuged. cccDNA was removed using a peristaltic pump. A bit more than equal volume of isopropyl alcohol saturated with water was added to cccDNA to remove ethidium bromide. The DNA sample was dialyzed against TE, resulting in about 3 ml of recombinant DNA. The recombinant plasmid containing a 4.6 kb DNA fragment was designated as pPCN1 (The recombinant plasmid containing a 4.7 kb DNA fragment was designated as pPCN3).

These plasmid DNAs were digested with EcoRI, BamHI, PstI, SacI and SalI. The restriction maps were constructed and are shown in Fig. 1.

### (7) DNA Sequencing

The location of a nitrile hydratase gene in the DNA fragments of pPCN1 and pPCN3 was determined according to the restriction map constructed and to the Southern hybridization method. Based on the results, the 2.456 kb BamHI-HincII DNA fragment was sequenced by the Sanger method [Sanger, F., Science 214: 1205-1210 (1981)] using M13 phage vector. The 2.456 kb DNA fragment from Pseudomonas chlororaphis B23 is shown in the Sequence Listing as defined by SEQ ID: No. 8.

All the nucleotide sequence deduced from the amino acid sequence as determined in (1) was found in the sequence as determined above. The sequence analysis also revealed that the DNA fragment contained the sequence coding for the α- and β-subunits.

### (8) Insertion of the BamHI-HincII DNA Fragment into an Expression Vector and Transformation

2.2 kb SphI-BamHI fragment of pPCN1 and 4.7 kb BamHI-SalI fragment of pPCN3 were cleaved and both fragments were ligated (Fig. 1). The ligated fragment was inserted into the SphI-SalI site of an expression vector pUC19 and the construct was designated as pPCN4.

E. coli JM105 was transformed with pPCN4 in a similar manner as in (5) and the transformant was designated as JM105/pPCN4 (FERM BP-2779).

### (9) Production of Nitrile Hydratase Using the Transformant and Conversion of Nitriles to Amides Using Nitrile Hydratase

JM105/pPCN4 was inoculated into 10 ml of 2×YT medium containing 50 µg/ml of ampicillin and incubated at 26.5°C overnight (12 hours). 100 µℓ of the resultant culture was added to 10 ml of 2×YT medium (50 µg/ml of ampicillin, 50 mg/ℓ of FeSO₄·7H₂O, 10 mg/ℓ of MgSO₄·7H₂O, 1 mg/ℓ of pyrroloquinolinequinone). The mixture was incubated at 26.5°C for five hours. IPTG was added to the mixture to a final concentration of 1 mM. The mixture was incubated at 26.5°C for 10 hours. After harvesting the cells, the cells were suspended in 5 ml of 1/20 M phosphate buffer (pH 7.7). 10 µℓ of the substrate solution (1M acrylonitrile) was added to 0.1 ml of the suspension. The mixture was incubated at 20°C for 20 minutes. As a control, the mixture obtained by the same procedure as described above but from E. coli JM105 was used. The reaction mixture was tested for the presences of acrylamide (the product of the enzymatic reaction) and acrylonitrile using HPLC. Acrylamide but no acrylonitrile was found in the reaction mixture of JM105/pPCN4, whereas acrylonitrile but no acrylamide was found in the reaction mixture of JM105.

### (1) INFORMATION FOR SEQ ID NO: 1

(i) SEQUENCE CHARACTERISTICS:
   (A) LENGTH: 200 amino acids
   (B) TYPE: Amino acid
   (C) STRANDEDNESS: -
   (D) TOPOLOGY: Linear
(ii) MOLECULE TYPE: Peptide
(vi) ORIGINAL SOURCE
   (A) ORGANISM: Pseudomonas chlororaphis
   (B) STRAIN: B23 (FERM BP-187)
(ix) FEATURES
   (A) OTHER INFORMATION
      α-subunit
(xi) SEQUENCE DESCRIPTION: SEQ ID NO: 1

### (2) INFORMATION FOR SEQ ID NO: 2

(i) SEQUENCE CHARACTERISTICS:
   (A) LENGTH: 220 amino acids
   (B) TYPE: Amino acid
   (C) STRANDEDNESS: -
   (D) TOPOLOGY: Linear
(ii) MOLECULE TYPE: Peptide
(vi) ORIGINAL SOURCE
   (A) ORGANISM: Pseudomonas chlororaphis
   (B) STRAIN: B23 (FERM BP-187)
(ix) FEATURES
   (A) OTHER INFORMATION
      β-subunit
(xi) SEQUENCE DESCRIPTION: SEQ ID NO: 2

### (3) INFORMATION FOR SEQ ID NO: 3

(i) SEQUENCE CHARACTERISTICS:
   (A) LENGTH: 600 base pairs
   (B) TYPE: Nucleic acid
   (C) STRANDEDNESS: Single
   (D) TOPOLOGY: Linear
(ii) MOLECULE TYPE: Genomic DNA
(vi) ORIGINAL SOURCE
   (A) ORGANISM: Pseudomonas chlororaphis
   (B) STRAIN: B23 (FERM BP-187)
(ix) FEATURES
   (A) OTHER INFORMATION
      α-subunit
(xi) SEQUENCE DESCRIPTION: SEQ ID NO: 3

### (4) INFORMATION FOR SEQ ID NO: 4

(i) SEQUENCE CHARACTERISTICS:
   (A) LENGTH: 660 base pairs
   (B) TYPE: Nucleic acid
   (C) STRANDEDNESS: Single
   (D) TOPOLOGY: Linear
(ii) MOLECULE TYPE: Genomic DNA
(vi) ORIGINAL SOURCE
   (A) ORGANISM: Pseudomonas chlororaphis
   (B) STRAIN: B23 (FERM BP-187)
(ix) FEATURES
   (A) OTHER INFORMATION
      β-subunit
(xi) SEQUENCE DESCRIPTION: SEQ ID NO: 4
   ATGGATGGCTTTCACGATCTCGGCGGTTTCCAAGGCTTTGGCAAA  45
   GTGCCGCACACCATCAACAGCCTCAGCTACAAACAGGTTTTCAAG  90
   CAGGACTGGGAACACCTGGCCTATAGCTTGATGTTTGTCGGCGTT  135
   GACCAATTGAAAAAGTTCAGCGTGGACGAAGTGCGTCATGCCGTC  180
   GAACGCCTGGACGTTCGCCAGCATGTCGGCACCCAGTACTACGAA  225
   CGCTACATCATCGCGACCGCCACGCTGCTGGTGGAAACGGGCGTT  270
   ATCACCCAGGCGGAGCTCGATCAGGCATTGGGTTCCCACTTCAAG  315
   CTGGCGAACCCCGCCCATGCGACAGGTCGCCCGGCGATCACCGGC  360
   AGGCCGCCCTTCGAAGTGGGCGATCGGGTTGTGGTTCGAGACGAA  405
   TATGTGGCGGGGCATATCCGCATGCCGGCCTACGTGCGCGGTAAG  450
   GAAGGCGTGGTCCTGCACCGCACCTCAGAGCAGTGGCCCTTCCCC  495
   GACGCCATTGGCCACGGCGACTTGAGCGCAGCCCATCAGCCTACC  540
   TACCACGTCGAGTTTCGCGTGAAAGATCTATGGGGTGACGCGGCA  585
   GATGACGGTTACGTCGTGGTCGATCTTTTCGAAAGCTACTTGGAT  630
   AAGGCCCCCGGTGCCCAAGCGGTGAACGCA

### (5) INFORMATION FOR SEQ ID NO: 5

(i) SEQUENCE CHARACTERISTICS:
   (A) LENGTH: 25 amino acids
   (B) TYPE: Amino acid
   (C) STRANDEDNESS: -
   (D) TOPOLOGY: Linear
(ii) MOLECULE TYPE: Peptide
(vi) ORIGINAL SOURCE
   (A) ORGANISM: Pseudomonas chlororaphis
   (B) STRAIN: B23 (FERM BP-187)
(ix) FEATURES
   (A) OTHER INFORMATION
      α-subunit: α₁
(xi) SEQUENCE DESCRIPTION: SEQ ID NO: 5

### (6) INFORMATION FOR SEQ ID NO: 6

(i) SEQUENCE CHARACTERISTICS:
   (A) LENGTH: 23 amino acids
   (B) TYPE: Amino acid
   (C) STRANDEDNESS: -
   (D) TOPOLOGY: Linear
(ii) MOLECULE TYPE: Peptide
(vi) ORIGINAL SOURCE
   (A) ORGANISM: Pseudomonas chlororaphis
   (B) STRAIN: B23 (FERM BP-187)
(ix) FEATURES
   (A) OTHER INFORMATION
      β-subunit: β₁
(xi) SEQUENCE DESCRIPTION: SEQ ID NO: 6

### (7) INFORMATION FOR SEQ ID NO: 7

(i) SEQUENCE CHARACTERISTICS:
   (A) LENGTH: 2070 base pairs
   (B) TYPE: Nucleic acid
   (C) STRANDEDNESS: Single
   (D) TOPOLOGY: Linear
(ii) MOLECULE TYPE: Genomic DNA
(vi) ORIGINAL SOURCE
   (A) ORGANISM: Rhodococcus
   (B) STRAIN: sp. N-774 (FERM BP-1936)
(ix) FEATURES
   from nucleotide No. 675 to 1295: subunit α
   from nucleotide No. 1325 to 1960: subunit β
(xi) SEQUENCE DESCRIPTION: SEQ ID NO: 7

### (8) INFORMATION FOR SEQ ID NO: 8

(i) SEQUENCE CHARACTERISTICS:
   (A) LENGTH: 2456 base pairs
   (B) TYPE: Nucleic acid
   (C) STRANDEDNESS: Single
   (D) TOPOLOGY: Linear
(ii) MOLECULE TYPE: Genomic DNA
(vi) ORIGINAL SOURCE
   (A) ORGANISM: Pseudomonas chlororaphis
   (B) STRAIN: B23 (FERM BP-187)
(ix) FEATURES
   from nucleotide No. 1021 to 1620: subunit a
   from nucleotide No. 1666 to 2325: subunit β
(xi) SEQUENCE DESCRIPTION: SEQ ID NO: 8

## Claims

1. A gene encoding *a* polypeptide having nitrile hydratase activity, said polypeptide comprising the α-subunit of the amino acid sequence as defined in the Sequence Listing by SEQ ID: No. 1 and the β-subunit of the amino acid sequence as defined in SEQ ID: No. 2.

2. The gene of claim 1 encoding α- and β-subunits, comprising the α-subunit encoding sequence as defined in the Sequence Listing by SEQ ID: No. 3, and the β-subunit-encoding sequence *as* defined in the Sequence Listing by SEQ ID: No. 4.

3. A recombinant DNA comprising the gene as claimed in claim 1 or 2.

4. A transformant transformed with the recombinant DNA of claim 3.

5. The transformant of claim 4 which is JM105/pPCN4 (FERM BP-2779).

6. A method of producing nitrile hydratase which comprises culturing the transformant as claimed in claim 4 or 5 and recovering nitrile hydratase from the culture.

7. A method of producing amides which comprises hydrating nitriles using nitrile hydratase obtained from the culture of the transformant of claim 4 or 5.

8. A method of producing amides which comprises culturing the transformant as claimed in claim 4 or 5, and hydrating nitriles to amides using the resultant culture, isolated bacterial cells, treated matter thereof, or a fixed material thereof.

## Patentansprüche

1. Gen, das ein Polypeptid mit Nitrilhydrataseaktivität codiert, wobei das Polypeptid die α-Untereinheit mit der Aminosäuresequenz umfaßt, wie sie in dem Sequenzproto-kolll durch SEQ ID: No. 1 definiert ist, und die β-Untereinheit mit der Aminosäuresequenz, wie sie in SEQ ID: No. 2 definiert ist.

2. Gen nach Anspruch 1, das α- und β-Untereinheiten codiert, umfassend die die α-Untereinheit codierende Sequenz, wie sie in dem Sequenzprotokoll durch SEQ ID: No. 3 definiert ist, und die die β-Untereinheit codierende Sequenz, wie sie in dem Sequenzprotokoll durch SEQ ID: No. 4 definiert ist.

3. Rekombinante DNA, umfassend das Gen nach Anspruch 1 oder 2.

4. Transformante, transformiert mit der rekombinanten DNA nach Anspruch 3.

5. Transformante nach Anspruch 4, die JM105/pPCN4 (FERM BP-2779) ist.

6. Verfahren zur Herstellung von Nitrilhydratase, umfassend das Züchten der Transformante nach Anspruch 4 oder 5 und die Gewinnung von Nitrilhydratase aus der Kultur.

7. Verfahren zur Herstellung von Amiden, umfassend die Hydratisierung von Nitrilen unter Verwendung einer aus der Kultur der Transformante nach Anspruch 4 oder 5 erhaltenen Nitrilhydratase.

8. Verfahren zur Herstellung von Amiden, umfassend das Züchten der Transformante nach Anspruch 4 oder 5 und das Hydratisieren von Nitrilen zu Amiden unter Verwendung der erhaltenen Kultur, isolierter bakterieller Zellen, behandelten Materials davon, oder eines fixierten Materials davon.

## Revendications

1. Gène codant un polypeptide qui possède une activité de nitrile hydratase et qui comporte une sous-unité *α*, dont la séquence d'acides aminés est définie dans le paragraphe "SEQ ID n°1" du chapitre "Liste de séquences", et une sous-unité β, dont la séquence d'acides aminés est définie dans le paragraphe "SEQ ID n°2" du chapitre "Liste de séquences".

2. Gène conforme à la revendication 1, qui code des sous-unités α et β et qui comporte la séquence définie dans le paragraphe "SEQ ID n°3" du chapitre "Liste de séquences", laquelle code la sous-unité *α*, et la séquence définie dans le paragraphe "SEQ ID n°4" du chapitre "Liste de séquences", laquelle code la sous-unité β.

3. ADN recombiné comportant un gène conforme à la revendication 1 ou 2.

4. Organisme transformé avec un ADN recombiné conforme à la revendication 3.

5. Organisme transformé conforme à la revendication 4, qui est JM 105/pPCN4 (FERM BP-2779).

6. Procédé de production d'une nitrile hydratase, qui comporte le fait de cultiver un organisme transformé, conforme à la revendication 4 ou 5, et le fait de récupérer la nitrile hydratase dans la culture.

7. Procédé de production d'amides, qui comporte le fait d'hydrater des nitriles au moyen d'une nitrile hydratase récupérée dans une culture d'un organisme transformé conforme à la revendication 4 ou 5.

8. Procédé de production d'amides, qui comporte le fait de cultiver un organisme transformé conforme à la revendication 4 ou 5, et le fait d'hydrater des nitriles en amides au moyen de la culture résultante, des cellules bactériennes isolées de celle-ci, ou d'un produit qui résulte de leur fixation ou d'un traitement qu'on leur a infligé.
